# EUROPEAN PATENT SPECIFICATION

(11) **EP 1 682 512 B1**
(45) Date of publication and mention of the grant of the patent: **31.03.2010**
(21) Application number: 04818346.1
(22) Date of filing: 04.11.2004
(51) Int. Cl.: G01N 33/50, G01N 33/574

(54) **IMMUNOHISTOCHEMICAL METHODS FOR MONITORING pERK LEVELS**
IMMUNOHISTOCHEMISCHE VERFAHREN ZUR ÜBERWACHUNG VON pERK SPIEGELN
METHODES IMMUNOHISTOCHIMIQUES DE CONTRÔLE DU NIVEAU pERK

(30) Priority: 04.11.2003 US 517495 P
(43) Date of publication of application: 26.07.2006
(73) Proprietor: Bayer Pharmaceuticals Corporation, West Haven, CT 06516 (US)
(72) Inventor: TAYLOR, Ian, Madison, Connecticut 06443 (US); WILHELM, Scott, Orange, Connecticut 06477 (US)
(74) Representative: Linkenheil, Dieter
(86) International application number: PCT/US2004/036977
(87) International publication number: WO 2005/044794

(56) References cited:
- US-A1- 2003 190 689
- NG S S W ET AL: "EFFECTS OF THE EPIDERMAL GROWTH FACTOR RECEPTOR INHIBITOR OSI-774, TARCEVA, ON DOWNSTREAM SIGNALING PATHWAYS AND APOPTOSIS IN HUMAN PANCREATIC ADENOCARCINOMA" MOLECULAR CANCER THERAPEUTICS, AMERICAN ASSOCIATION OF CANCER RESEARCH, US, vol. 1, no. 10, August 2002 (2002-08), pages 777-783, XP009056210 ISSN: 1535-7163
- RICCIARDI MARIA R ET AL: "Flow Cytometric Monitoring of Constitutive MAPK Phosphorylation and of Pharmacodynamic MEK/MAPK Inhibition in Primary AML Samples." BLOOD, vol. 100, no. 11, 16 November 2002 (2002-11-16), page Abstract No. 4610, XP009094998 & 44TH ANNUAL MEETING OF THE AMERICAN SOCIETY OF HEMATOLOGY; PHILADELPHIA, PA, USA; DECEMBER 06-10, 2002 ISSN: 0006-4971
- AGUIRRE-GHISO JULIO A ET AL: "ERKMAPK activity as a determinant of tumor growth and dormancy; Regulation by p38SAPK." CANCER RESEARCH, vol. 63, no. 7, 1 April 2003 (2003-04-01), pages 1684-1695, XP009094969 ISSN: 0008-5472
- ZHU JIAN-HUI ET AL: "Cytoplasmic aggregates of phosphorylated extracellular signal-regulated protein kinases in Lewy body diseases." AMERICAN JOURNAL OF PATHOLOGY, vol. 161, no. 6, December 2002 (2002-12), pages 2087-2098, XP002466466 ISSN: 0002-9440
- JERGENSEN ET AL.: 'Expression of Activated Extracellular Signal-Regulated kinases 1/2 in Malignant Melanomas: Relationship with Clinical Outcome' CLINICAL CANCER RESEARCH vol. 9, no. 14, 01 November 2003, pages 5325 - 5331, XP003005791
- TANIMURA ET AL.: 'Specific blockade of th ERK pathway inhibits the invasiveness of tumor cells: down-regulation of matrix metalloproteinase-3/-9/-14 and CD44' BIOCHEMICAL AND BIPHYSICAL RESEARCH COMMUNICATIONS vol. 304, no. 4, 16 May 2003, pages 801 - 806, XP003005792
- ADEYINKA ET AL.: 'Activated Mitogen-activated Protein Kinase Expression during Human Breast Tumorigenesis and Breast Cancer Progression' CLINICAL CANCER RESEARCH vol. 8, no. 6, June 2002, pages 1747 - 1753, XP003005793
- FU ET AL.: 'Effects of Raf-Kinase Inhibitor Protein Expression on Suppression of Prostae Cancer Metastasis' JOURNAL OF THE NATIONAL CANCER INSTITUTE vol. 95, 18 June 2003, pages 878 - 879, XP003005794

## Description

### FIELD OF THE INVENTION

The present invention relates to immunohistochemical methods to identify biomarkers. For example, this invention relates to the use of phospho-ERK (pERK) levels in tumor cells as a biomarker for a Raf kinase inhibitor.

### BACKGROUND OF THE INVENTION

Many disease states are characterized by differences in the phosphorylation levels of various signaling proteins through changes in the activity of protein kinases or phosphatases. Compounds which are used as therapeutics to treat these various diseases (e.g., cancer) presumably reverse some or all of these protein phosphorylation level modulations. The phosphorylation level or change in level of at least some of these proteins may, therefore, be used as a method to monitor, or even predict, the efficacy of such therapeutics. As a result, some or all of these protein phosphorylation levels or changes in level can be considered to be, and can be utilized as, a biomarker. Besides being used to monitor or predict the efficacy of a therapeutic, biomarkers might also be used to select patients who are predicted to respond positively to therapeutic administration and those that might revert to non-responsive status. The analysis of these protein phosphorylation levels or changes in level may be performed in the target tissue of interest (e.g., tumor) or in some surrogate cell population (e.g., peripheral blood leukocytes). In the latter case, correlation of the protein phosphorylation levels or changes in level with efficacy (e.g., tumor shrinkage or non-growth) should be especially strong for the expression change pattern to be used as a marker for efficacy.

Raf kinase is a protein involved in the Ras signal transduction pathway. Ras regulates several pathways which synergistically induce cellular transformation, including the Raf/Mek/Erk cascade and the rac and rho pathways. In particular, Ras activates the Raf/MEK pathway by first localizing Raf to the plasma membrane, where Raf initiates a mitogenic kinase cascade (Hall, Science 264:1413-1414, 1994). Activated Raf phosphorylates and activates MEK (a known downstream substrate), which in turn phosphorylates and activates ERK. Activated ERK then translocates from the cytoplasm into the nucleus and modulates gene expression via the phosphorylation of transcription factors. Thus, activation of Raf kinase, via activation of Ras, is considered an important mechanism by which human cancer develops.

A number of studies have suggested that inhibition of Raf kinase is an important target for cancer therapy. For instance, dominant negative mutants of Raf, MEK, or ERK activity significantly reduce the transforming ability of mutant Ras in a rodent fibroblast background. Moreover, human tumor cell lines expressing a dominant negative MEK were deficient in their ability to grow both in tissue culture as well as in anchorage independent growth assays when compared to the parental cell line. Such mutants also inhibited both the primary and metastatic growth of human tumor xenografts *in vivo.* Additional support for targeting Raf kinase comes from work with antisense oligonucleotides. ISIS 5132, a phosphorothioate antisense oligonucleotide designed to target c-Raf, was found to inhibit the growth of the A549 human lung xenograft *in vivo* (Monia, et al., Proc. Natl. Acad. Sci 93:15481-15484, 1996). When taken together these data suggest that the Raf kinase protein is a significant contributor to the malignant phenotype driven by activated ras signaling. Moreover, the data also suggests that small molecule inhibitors of raf kinase activity will be an important therapeutic mechanism in the treatment of cancer.

However, due to the network of growth factor receptors, ligands, and downstream cell proliferation and cell survival effector molecules, inhibiting specific kinases may not be an effective therapeutic strategy in all individuals with cancer, as various compensatory pathways may exist to overcome the therapeutic inhibition. Accordingly, it will be useful to identify biological markers that indicate, in an individual patient, whether the patient's tumor is responding to a particular therapeutic intervention. While tumor size or progression of disease has traditionally been used to determine whether an individual was responding to a particular therapy, use of molecular markers may allow earlier identification of responders and non-responders. Non-responders may then be offered alternate therapy, and spared potential side effects of a therapy that is ineffective for their specific tumor.

It would be useful to identify one or more or a combination of molecular markers capable of indicating whether an individual's tumor is responding to treatment (e.g., treatment with a Raf kinase inhibitor). Such markers would help (i) to identify in which clinical settings and patient populations the therapeutic approach is most likely to be effective and (ii) to assess, in individual patients, whether the patient's tumor is responding to a specific treatment.

Because normal tissue has a characteristic appearance, histologic examination is often utilized to identify diseased tissue. Immunohistochemistry (IHC) is a useful tool in histological diagnosis. IHC may be utilized to detect an entity in a sample by using specific binding agents capable of binding to the detectable entity. The specific binding agent may comprise an antibody, and the detectable entity may comprise a polypeptide, protein, or epitope. The increasing availability of such antibodies may help in differential diagnosis of diseased and normal tissue. IHC methods are described in detail in Harlow and Lane (Antibodies: A Laboratory Manual).

IHC techniques require a series of steps which may be conducted on a tissue section mounted on a glass slide or other planar support. Certain morphological indicators of disease states may be highlighted by selective staining. For example, a sample is taken from a patient, and then fixed and exposed to antibodies against an antigen of interest. Further processing steps, for example, antigen retrieval, exposure to secondary antibodies (usually coupled to a suitable enzyme) and to chromogenic enzyme substrates, may be necessary to reveal the pattern of antigen binding.

In general, there are two categories of histological materials: (a) preparations comprising fresh tissues and/or cells, which are not fixed with aldehyde-based fixatives, and (b) fixed and embedded tissue specimens, often archive material. Many methods for fixing and embedding tissue specimens are known (e.g., alcohol fixation). However, the most widely used fixing/embedding technique employs formalin-fixation and subsequent paraffin embedding (FFPE). A typical FFPE IHC staining procedure may involve the steps of: cutting and trimming tissue, fixation, dehydration, paraffin infiltration, cutting in thin sections, mounting onto glass slides, baking, deparaffination, rehydration, antigen retrieval, blocking steps, applying primary antibody, washing, applying secondary antibody-enzyme conjugate, washing, applying enzyme chromogen substrate, washing, counter staining, cover slipping, and microscope examination.

As described above, phosphorylation of ERK is downstream of the ras/raf/MEK/ERK pathway. In renal cell carcinoma (RCC), increased levels of phosphorylated (i.e., activated) raf and ERK proteins are present approximately 50% of the time (Oka, et al., Cancer Res. 55:4182-4187, 1995). As such, patients with elevated phosphorylated ERK may be more likely to benefit from treatment with a Raf kinase inhibitor than patients whose ras/raf/ERK pathway is not upregulated. In order to assess this effect, a tumor biopsy sample may be analyzed for pERK levels using quantitative or semi-quantitative immunohistochemistry.

### SUMMARY OF THE INVENTION

The present invention is directed to immunohistochemical methods to identify biomarkers. For example, this invention relates to the use of phospho-ERK (pERK) levels in tumor cells as a biomarker for a Raf kinase inhibitor.

One embodiment of the present invention is a method of assessing in a subject needing treatment for a tumor, whether the subject is likely to exhibit a favorable clinical response to such treatment via a method that comprises determining the pre-treatment level of pERK in the tumor.

Another embodiment of the present invention is a method of assessing in a subject in need of treatment for a tumor, whether the subject is likely to exhibit a favorable clinical response to such treatment by determining the pre-treatment level of pERK in the tumor, administering a therapeutically effective amount of a Raf kinase inhibitor and determining the level of pERK in the tumor after an initial period of treatment with the therapeutic agent. A decrease in the pERK. level indicates that the subject is more likely to exhibit a favorable clinical response to the treatment, compared to a subject with no change or an increase in pERK levels.

Another aspect of the present invention is a method for screening the effects of a drug, for example, a Raf kinase inhibitor, on a tissue or cell sample comprising the step of analyzing pERK levels in tumor cells, wherein pERK levels in tumor cells in the tissue or cell sample are analyzed before and after exposure to the drug, and a variation in the pERK levels in tumor cells is indicative of a drug effect or provides a patient diagnosis or predicts a patient's response to the treatment.

A further aspect of the present invention is a method for distinguishing between normal and disease states comprising the step of analyzing pERK levels in tumor cells, wherein the pERK levels of normal and disease tissues are analyzed, and a variation in pERK levels in tumor cells is indicative of a disease state.

The present invention also provides a method for providing a patient diagnosis comprising the step of analyzing pERK levels in tumor cells, wherein the pERK levels of normal and patient samples are analyzed, and a variation in the pERK levels in tumor cells in the patient sample is diagnostic of a disease. The patient samples include, but are not limited to, blood, amniotic fluid, plasma, semen, bone marrow, and tissue biopsy.

The invention also provides a method for patient selection comprising the step of analyzing pERK levels in tumor cells, wherein the pERK levels of patient samples are analyzed, and the pERK levels in tumor cells in the patient sample is prognostic of that patient's response to a Raf kinase inhibitor. The patient samples include, but are not limited to, blood, amniotic fluid, plasma, semen, bone marrow, and tissue biopsy. The levels of pERK in tumor cells may then be considered a biomarker for efficacy of a Raf kinase inhibitor.

Another aspect of the present invention is a method for discovering novel drugs comprising the step of analyzing pERK levels in tumor cells, wherein the pERK levels are analyzed before and after exposure to the drug, and a variation in the pERK levels in tumor cells is indicative of drug efficacy. The levels of pERK in tumor cells may then be considered a biomarker for efficacy of a Raf kinase inhibitor.

Another embodiment of the present invention is a diagnostic kit. For example, the diagnostic kit may contain photomicrographs of different sets of cells at various levels of staining. The kit may also contain an indication that a particular set of cells represents the cut-off or threshold point, with cells matching or exceeding that staining being "positive," and those cells having less staining being "negative." The kits may also contain actual samples of tissues or cells, which are already stained, on control slides. For example, a kit may include several reference slides with sections of breast carcinoma cell lines that represent different levels of a breast specific protein expression.

### DESCRIPTION OF THE DRAWINGS

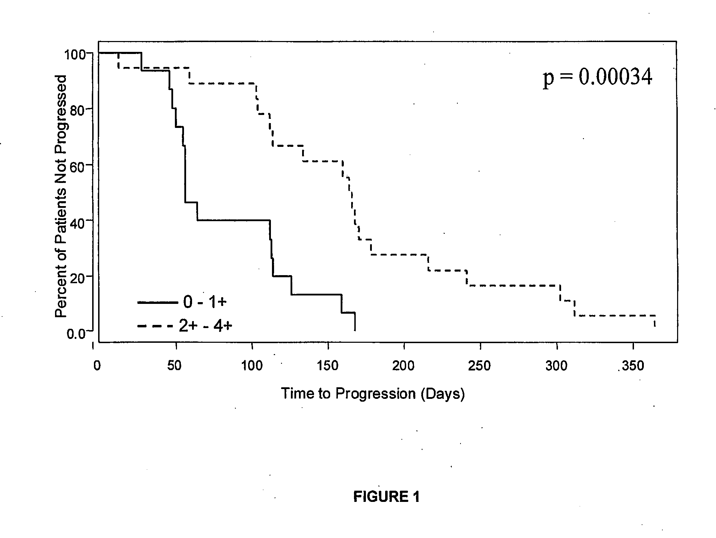

***Figure 1******.*** Kaplan-Meier plot of percentage of patients not progressed as a function of time to progression in patients with a maximum pERK staining intensity of either 0 and 1+ or 2 through 4+.

### DESCRIPTION OF THE INVENTION

It is to be understood that this invention is not limited to the particular methodology, protocols, cell lines, animal species or genera, constructs, and reagents described and as such may vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a," "and," and "the" include plural reference unless the context clearly dictates otherwise. Thus, for example, reference to "a protein" is a reference to one or more proteins and includes equivalents thereof known to those skilled in the art, and so forth.

Unless defined otherwise, all technical and scientific terms used herein have the same meaning as commonly understood to one of ordinary skill in the art to which this invention belongs. Any methods, devices, and materials similar or equivalent to those described herein can be used in the practice or testing of the invention.

### Definitions

For convenience, the meaning of certain terms and phrases employed in the specification, examples, and appended claims are provided below.

The phrase "a corresponding normal cell of" or "normal cell corresponding to" or "normal counterpart cell of" a diseased cell refers to a normal cell of the same type as that of the diseased cell. For example, a corresponding normal cell of a B lymphoma cell is a B cell.

The term "agonist," as used herein, is meant to refer to an agent that mimics or up-regulates (e.g., potentiates or supplements) the bioactivity of a protein. An agonist may be a wild-type protein or derivative thereof having at least one bioactivity of the wild-type protein. An agonist may also be a compound that up-regulates expression of a gene or which increases at least one bioactivity of a protein. An agonist can also be a compound which increases the interaction of a polypeptide with another molecule, for example, a target peptide or nucleic acid.

"Antagonist," as used herein, is meant to refer to an agent that down-regulates (e.g., suppresses or inhibits) at least one bioactivity of a protein. For example, a Raf kinase inhibitor is an example of such an antagonist. An antagonist may be a compound which inhibits or decreases the interaction between a protein and another molecule, for example, a target peptide or enzyme substrate. An antagonist may also be a compound that down-regulates expression of a gene or which reduces the amount of expressed protein present.

The term "antibody," as used herein, is intended to include whole antibodies, for example, of any isotype (IgG, IgA, IgM, IgE, etc.), and includes fragments thereof which are also specifically reactive with a vertebrate (e.g., mammalian) protein. Antibodies may be fragmented using conventional techniques and the fragments screened for utility in the same manner as described above for whole antibodies. Thus, the term includes segments of proteolytically-cleaved or recombinantly-prepared portions of an antibody molecule that are capable of selectively reacting with a certain protein. Non-limiting examples of such proteolytic and/or recombinant fragments include Fab, F(ab')2, Fab', Fv, and single chain antibodies (scFv) containing a V[L] and/or V[H] domain joined by a peptide linker. The scFv's may be covalently or non-covalently linked to form antibodies having two or more binding sites. The subject invention includes polyclonal, monoclonal, humanized, or other purified preparations of antibodies and recombinant antibodies.

"Biological activity," "bioactivity," "activity," or "biological function," which are used interchangeably, herein mean an effector or antigenic function that is directly or indirectly performed by a polypeptide (whether in its native or denatured conformation), or by any subsequence thereof. Biological activities include binding to polypeptides, binding to other proteins or molecules, activity as a DNA binding protein, as a transcription regulator, ability to bind damaged DNA, etc. A bioactivity can be modulated by directly affecting the subject polypeptide. Alternatively, a bioactivity can be altered by modulating the level of the polypeptide, such as by modulating expression of the corresponding gene.

The term "biological sample", as used herein, refers to a sample obtained from an organism or from components (e.g., cells) of an organism. The sample may be of any biological tissue or fluid. The sample may be a "clinical sample" which is a sample derived from a patient. Such samples include, but are not limited to, sputum, blood, blood cells (e.g., white cells), tissue or fine needle biopsy samples, urine, peritoneal fluid, and pleural fluid, or cells therefrom. Biological samples may also include sections of tissues such as frozen sections taken for histological purposes.

The term "biomarker" or "marker" encompasses a broad range of intra- and extra-cellular events as well as whole-organism physiological changes. Biomarkers may be represent essentially any aspect of cell function, for example, but not limited to, levels or rate of production of signaling molecules, transcription factors, metabolites, gene transcripts as well as post-translational modifications of proteins. Biomarkers may include whole genome analysis of transcript levels or whole proteome analysis of protein levels and/or modifications.

The term "biomarker" refers to a gene or gene product (e.g., protein) which is up- or down-regulated in a compound-treated, diseased cell of a subject having the disease relative to a counterpart untreated diseased cell. That is, the gene or gene product is sufficiently specific to the treated cell that it may be used, optionally with other genes or gene products, to identify, predict or detect efficacy of a small molecule for the disease. Generally, a biomarker is a gene or gene product that is characteristic of efficacy of a compound in a diseased cell or the response of that diseased cell to treatment by the compound.

The term "cancer" includes, but is not limited to, solid tumors, such as cancers of the breast, respiratory tract, brain, reproductive organs, digestive tract, urinary tract, eye, liver, skin, head and neck, thyroid, parathyroid, and their distant metastases. The term also includes lymphomas, sarcomas, and leukemias.

Examples of breast cancer include, but are not limited to, invasive ductal carcinoma, invasive lobular carcinoma, ductal carcinoma *in situ,* and lobular carcinoma *in situ.*

Examples of cancers of the respiratory tract include, but are not limited to, small-cell and non-small-cell lung carcinoma, as well as bronchial adenoma and pleuropulmonary blastoma.

Examples of brain cancers include, but are not limited to, brain stem and hypophtalmic glioma, cerebellar and cerebral astrocytoma, medulloblastoma, ependymoma, as well as neuroectodermal and pineal tumor.

Tumors of the male reproductive organs include, but are not limited to, prostate and testicular cancer. Tumors of the female reproductive organs include, but are not limited to, endometrial, cervical, ovarian, vaginal, and vulvar cancer, as well as sarcoma of the uterus.

Tumors of the digestive tract include, but are not limited to, anal, colon, colorectal, esophageal, gallbladder, gastric, pancreatic, rectal, small-intestine, and salivary gland cancers.

Tumors of the urinary tract include, but are not limited to, bladder, penile, kidney, renal pelvis, ureter, and urethral cancers.

Eye cancers include, but are not limited to, intraocular melanoma and retinoblastoma.

Examples of liver cancers include, but are not limited to, hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to, squamous cell carcinoma, Kaposi's sarcoma, malignant melanoma, Merkel cell skin cancer, and non-melanoma skin cancer.

Head-and-neck cancers include, but are not limited to, laryngeal / hypopharyngeal / nasopharyngeal / oropharyngeal cancer, and lip and oral cavity cancer.

Lymphomas include, but are not limited to, AIDS-related lymphoma, non-Hodgkin's lymphoma, cutaneous T-cell lymphoma, Hodgkin's disease, and lymphoma of the central nervous system.

Sarcomas include, but are not limited to, sarcoma of the soft tissue, osteosarcoma, malignant fibrous histiocytoma, lymphosarcoma, and rhabdomyosarcoma.

Leukemias include, but are not limited to, acute myeloid leukemia, acute lymphoblastic leukemia, chronic lymphocytic leukemia, chronic myelogenous leukemia, and hairy cell leukemia.

"A diseased cell of cancer" refers to a cell present in subjects having cancer. That is, a cell which is a modified form of a normal cell and is not present in a subject not having cancer, or a cell which is present in significantly higher or lower numbers in subjects having cancer relative to subjects not having cancer.

The term "gene" refers to a nucleic acid sequence that comprises control and coding sequences necessary for the production of a polypeptide or precursor. The polypeptide can be encoded by a full length coding sequence or by any portion of the coding sequence. The gene may be derived in whole or in part from any source known to the art, including a plant, a fungus, an animal, a bacterial genome or episome, eukaryotic, nuclear or plasmid DNA, cDNA, viral DNA, or chemically synthesized DNA. A gene may contain one or more modifications in either the coding or the untranslated regions which could affect the biological activity or the chemical structure of the expression product, the rate of expression, or the manner of expression control. Such modifications include, but are not limited to, mutations, insertions, deletions, and substitutions of one or more nucleotides. The gene may constitute an uninterrupted coding sequence or it may include one or more introns, bound by the appropriate splice junctions.

As used herein, the terms "label" and "detectable label" refer to a molecule capable of detection, including, but not limited to, radioactive isotopes, fluorophores, chemiluminescent moieties, enzymes, enzyme substrates, enzyme cofactors, enzyme inhibitors, dyes, metal ions, ligands (e.g., biotin or haptens), and the like. The term "fluorescer" refers to a substance or a portion thereof which is capable of exhibiting fluorescence in the detectable range. Examples of labels which may be used in the present invention include fluorescein, rhodamine, dansyl, umbelliferone, Texas red, luminol, NADPH, alpha - beta -galactosidase, and horseradish peroxidase.

The term "patient" or "subject" as used herein includes mammals (e.g., humans and animals).

The "profile" of a cell's biological state refers to the levels of various constituents of a cell that are known to change in response to drug treatments and other perturbations of the biological state of the cell. Constituents of a cell include, for example, levels of RNA, levels of protein or protein activity, or phosphorylation levels.

The term "protein," "polypeptide," and "peptide" are used interchangeably herein when referring to a gene product.

"Small molecule," as used herein, refers to a composition with a molecular weight of less than about 5 kD and most preferably less than about 4 kD. Small molecules can be nucleic acids, peptides, polypeptides, peptidomimetics, carbohydrates, lipids, or other organic or inorganic molecules. Many pharmaceutical companies have extensive libraries of chemical and/or biological mixtures, often fungal, bacterial, or algal extracts, which can be screened with any of the assays of the invention to identify compounds that modulate a bioactivity.

A "variant" of polypeptide refers to a polypeptide having an amino acid sequence in which one or more amino acid residues is altered. The variant may have "conservative" changes, wherein a substituted amino acid has similar structural or chemical properties (e.g., replacement of leucine with isoleucine). A variant may also have "nonconservative" changes (e.g., replacement of glycine with tryptophan). Analogous minor variations may include amino acid deletions or insertions, or both. Guidance in determining which amino acid residues may be substituted, inserted, or deleted without abolishing biological or immunological activity may be identified using computer programs well known in the art, for example, LASERGENE software (DNASTAR).

The present invention relates to the use of phospho-ERK (pERK) levels in tumor cells as a biomarker for a Raf kinase inhibitor (see, e.g., Wilhelm, et al., Cancer Res. 64:7099-7109, 2004, which is incorporated herein by reference in its entirety).

The methods of the present invention are directed to the use of biomarkers to monitor a subject's response to a therapeutic treatment, that is, to determine whether the subject is likely to have a favorable clinical response to that treatment. The methods of the present invention are directed to monitoring changes in levels of biomarkers in the early period of therapeutic treatment of a tumor with a Raf kinase inhibitor, and to identify subjects who are likely to exhibit a favorable clinical response to such treatment (compared to the likelihood of such a response in the general population).

The methods of the present invention are also directed to the use of levels of biomarkers prior to initiating therapy, as an aid in predicting whether the subject will have a favorable clinical response to a specified therapeutic treatment. The methods of the present invention are further directed to determining levels of biomarkers prior to therapeutic treatment of a tumor with a Raf kinase inhibitor, and to identify subjects who are likely to respond favorably (clinically) to such treatment (compared to the likelihood of such a response in the general population). As used herein, predictive is not meant to imply a 100% predictive ability, but to indicate that subjects with certain characteristics are more likely to experience a favorable clinical response than subjects who lack such characteristics. However, as will be apparent to one skilled in the art, some individuals identified as more likely to experience a favorable clinical response will nonetheless experience progression of disease. It will further be apparent to one skilled in the art that, just as certain conditions are identified herein as associated with an increased likelihood of a favorable clinical response, the absence of such conditions will be associated with a decreased likelihood of a favorable clinical response.

As used herein, a favorable response (or favorable clinical response) to a treatment refers to a biological or physical response that is recognized by those skilled in the art as indicating a decreased rate of tumor growth compared to tumor growth that would occur in the absence of any treatment. Favorable clinical response as used herein is not meant to indicate a cure. A favorable clinical response to therapy may include a lessening of symptoms experienced by the subject, an increase in the expected or achieved survival time, a decreased rate of tumor growth, cessation of tumor growth (stable disease), and/or regression of the tumor mass (each as compared to that which would occur in the absence of therapy).

As is well known in the art, tumors are frequently metastatic in that a first (primary) locus of tumor growth spreads to one or more anatomically separate sites. As used herein, reference to a tumor in a subject includes not only the primary tumor, but metastatic tumor growth as well. In some cases, the primary tumor may be surgically inaccessible while metastases are more readily accessible.

### Biological markers in clinical medicine

The identification of tumor characteristics or biomolecules that may be utilized as surrogate markers to predict the clinical response of an individual patient to a particular treatment (medicine response markers) and to identify those subjects most likely to respond favorably to a given treatment as well as those who are not likely to respond (and who should thus be considered for alternative treatments) will be of assistance in clinical practice. Additionally, such markers may be used in clinical trials to identify groups of patients that respond (or do not respond) to a particular therapy and to identify traits and phenotypes common to responders and non-responders.

Anderson, et al., (Ins. J. Cancer 94:774, 2001) report that the EGFR tyrosine kinase inhibitor ZD1839 inhibited the proliferation of human cancer cell lines both in vitro and in vivo. Furthermore, reductions in tumor growth rates (SKOV3 and MDA-MB-231 xenografts in mice) were reported to coincide with inhibition of constitutive ERK MAP kinase activation. Preincubation of cells with ZD1839 was also reported to block EGF-induced increases in activation of ERK MAP kinases and PKB/Akt in SKOV (human ovarian cancer) cells.

Albanell, et al., (Semin. Oncol. 28(5Suppl. 16):56-66, 2001) report that administration of ZD 1839 to humans in clinical trials resulted in decreased expression of both activated MAPK and Ki-67 in keratinocytes (assessed in biopsies of normal skin taken prior to and following ZD1839 administration; the basal layer of epidermis has high levels of EGF receptor expression). Albanell, et al., (J. Clin. Oncol. 20:4292-4302, 2002) also reported that serial skin biopsies taken before treatment and at approximately day 28 of treatment indicated inhibition of the EGFR signaling pathway.

Albanell, et al., (Cancer Res. 61:6500-6510, 2001) reported that in patients treated with a chimeric anti-EGF receptor antibody (Cetuximab; C225), activation of ERK1 and ERK2 in skin was lower compared to control (non-patient) skin.

It has been reported that ERK1 and ERK2 (ERK1/2) can prime Estrogen Receptor (ER) signalling via phosphorylation of the ER, and that exaggerated ERK1/2 MAPK activity might be capable of driving ER signaling and thus, tumor growth in the absence of estrogen (phenotypically evidenced as hormone resistance in the tumor) (*see, e.g.,* Coutts & Murphy, Cancer Res. 58:4071-4074, 1998). Gee, et al., (Ins. J. Cancer (Pred. Oncol) 95:247-254, 2001) reported that in a collection of breast cancer tissue samples, pMAPK positive status was found in 83% of ER negative breast cancers; and that increased ERK1/2 MAPK phosphorylation was associated with earlier relapse and reduced survival time in both ER negative and ER positive disease.

Most mitogenic signals transduced through growth factor receptor activation ultimately converge on a common downstream effecter, ERK1/2 MAP kinase (Egan and Weinberg, Nature 365:781-783,1993). Activated ERK1/2 serves as a transcription factor regulating tumor cell proliferation and survival (Pulverer, et al., Nature 353:670-674, 1991). Increased expression of activated ERK1/2 has also been demonstrated in a number of human malignancies (Hoshino, et al., Oncogene 18:813-822, 1999); Albanell, et al., Cancer Res. 61:6500-6510, 2001).

The present invention correlates the clinical effect of a Raf kinase inhibitor with baseline levels of pERK in tumor biopsies. That is, in patients, higher baseline levels of the particular molecular marker in tumor tissue were correlated with a patient's clinical response to anti-tumor therapy such as a Raf kinase inhibitor.

The present invention provides a method of screening subjects treated with a Raf kinase inhibitor for a tumor and to identify those subjects who are most likely to respond favorably to the treatment. As such, identification of patients who respond to treatment may serve as an aid in clinical decision-making.

In one embodiment of the present invention, one or more cells from the subject to be tested are obtained and fixed for processing for immunohistochemical analysis. For example, a tumor biopsy or sample of peripheral blood leukocytes (PBLs) cells may be obtained from the subject. It is also possible to obtain a cell sample from a subject, and then to enrich the sample for a desired cell type. For example, cells may be isolated from other cells using a variety of techniques, such as isolation with an antibody binding to an epitope on the cell surface of the desired cell type. Where the desired cells are in a solid tissue, particular cells may be dissected, for example, by microdissection or by laser capture microdissection (LCM) (*see, e.g.,* Bonner, et al., Science 278:1481, 1997; Emmert-Buck, et al., Science 274:998, 1996; Fend, et al., Am. J. Path. 154:61, 1999; and Murakami, et al., Kidney Int. 5 8:1346, 2000).

The methods of the present invention comprise determining the pre-treatment and initial treatment levels of a biological marker in a subject's tumor. Any suitable method of determining the level of specific biological marker may be utilized in the present methods. One such method involves obtaining a biopsy sample or cell aspirate from the subject's tumor and assessing marker levels by any suitable means, as would be apparent to one skilled in the art. The pre-treatment sample may be tumor tissue that was surgically excised as part of the treatment plan, or may be a biopsy done solely for determination of marker levels. The tissue sample must be processed in a manner that allows accurate detection of phosphorylated proteins. For example, if the tissue sample is paraffin-embedded, it may be fixed in the presence of phosphatase inhibitors and in a neutralized buffered formalin solution.

According to the methods of the present invention, the pre-treatment level of a specified marker or markers.in the subject's tumor tissue may be assessed before the subject begins a course of therapeutic treatment. For example, the assessment may be performed within about three weeks prior to treatment, within about two weeks prior to treatment, or within about one week or less prior is to treatment.

After an initial treatment period has passed, the same marker or markers may be reassessed to determine alterations in the level of this marker or markers. A decrease in pERK may indicate that the subject is more likely to respond favorably to treatment with a Raf kinase inhibitor as compared to a similar subject with unchanged or increased levels of this marker. As an example, the subject may exhibit at least about a 30% decrease in pERK level or the decrease in pERK index (or comparable measure) may be at least about 50%, 70%, 80%, or greater.

The present methods are suitable for use in subjects undergoing their first course of treatment, or subjects who have previously received a course of treatment for a tumor.

In the methods of the present invention, the levels of biological markers may be assessed pre-treatment, and may be reassessed at some point during treatment (e.g., following an initial treatment period). Reassessment of marker levels may occur at a time when the therapeutic agent has physically reached the site of the tumor for a period sufficient to allow a biological response to the therapeutic agent in the tumor tissue. In one embodiment of the present invention, the initial treatment period may be the period of time required for the therapeutic agent to reach steady-state plasma concentration (or shortly thereafter). The reassessment of biological markers may also occur shortly after the initial treatment period and prior to the end of a course of therapy such that therapy may be discontinued in subjects who are not likely to respond. Furthermore, reassessment may also be conducted at or immediately following the end of a course of therapy to determine if the subject would be suitable for a second course of the same therapy, if necessary.

Any suitable method of detecting specific biological markers may be used in the present methods. For example, immunohistochemistry (IHC) may be utilized. This staining method is based on immunoenzymatic reactions using monoclonal or polyclonal antibodies to detect cells or specific proteins such as tissue antigens. Typically, immunohistochemistry protocols include detection systems that allow visualization of the markers (e.g., via light microscopy or an automated scanning system) for qualitative or quantitative analyses. Various immunoenzymatic staining methods are known in the art for detecting a protein of interest. For example, immunoenzymatic interactions may be visualized using different enzymes such as peroxidase, alkaline phosphatase, or different chromogens such as DAB, AEC, or Fast Red.

Briefly, a primary antibody that recognizes the target molecule of interest is introduced to a pathological specimen. The primary antibody binds to the target molecule in or on the pathological specimen. After incubation, a wash is performed to remove unbound antibody. Then, a secondary antibody directed against the primary antibody and labeled with an enzyme, is incubated with the pathological specimen. During incubation, the secondary antibody will bind to the primary antibody. Alternatively, the primary antibody, which recognizes the target molecule, is labeled with the enzyme and a secondary antibody is not necessary. In another example, the labeled antibody may be labeled with biotin rather than an enzyme. Then, in an additional step, enzyme-labeled avidin or streptavidin is introduced to the sample and allowed to bind to the biotinylated antibody.

IHC has been used in a wide variety of immunodiagnostic applications, such as serodiagnostics to detect antigens from a wide range of specific viruses, bacteria, fungi and parasites, and to measure the presence of antibodies against these microorganisms. Similarly, these techniques may be utilized to monitor, for example, hormone levels, hematological factors, serum tumor markers, drug levels, or antibody levels. In addition, IHC may be used to classify and diagnose poorly differentiated malignant tumors. Further, IHC permits the identification of the primary site or origin of metastatic tumors, as tumors usually contain markers which identify the site of origin.

The methods of the present invention may be accomplished using any suitable method or system of immunohistochemistry, as will be apparent to one skilled in the art, including automated systems, quantitative IHC, semi-quantitative IHC, and manual methods.

As used herein, quantitative immunohistochemistry refers to an automated method of scanning and scoring samples that have undergone immunohistochemistry to identify and quantitate the presence of a specified biomarker such as an antigen or other protein. The score given to the sample may be a numerical representation of the intensity of the immunohistochemical staining of the sample, and represents the amount of target biomarkers present in the sample. For example, Optical Density (OD) is a numerical score that represents intensity of staining. As used herein, semi-quantitative immunohistochemistry refers to scoring of immunohistochemical results by the human eye, where a trained operator ranks results numerically (e.g., as 1+, 2+, or 3+).

Various automated sample processing, scanning, and analysis systems suitable for use with immunohistochemistry are available in the art. Such systems may include automated staining (e.g,. the Benchmark system, Ventana Medical Systems, Inc.) and microscopic scanning, computerized image analysis, serial section comparison (to control for variation in the orientation and size of a sample), digital report generation, and archiving and tracking of samples (such as slides on which tissue sections are placed). Cellular imaging systems are commercially available that combine conventional light microscopes with digital image processing systems to perform quantitative analysis on cells and tissues, including immunostained samples (e.g., CAS-200 system, Becton, Dickinson & Co.).

### Data Analysis Methods

Descriptions relating to the pattern or distribution of staining may be included to aid the analysis. For example, Score 0 (negative): no staining is observed, or membrane staining is observed in less than 10% of tumor cells. Score 1+ (negative): a faint or barely perceptible membrane staining is detected in more than 10% of the tumor cells. The cells are only stained in part of the membrane. Score 2+ (weakly positive): a weak to moderate complete membrane staining is observed in more than 10% of the tumor cells. Score 3+ (strongly positive): a strong complete membrane staining is observed in more than 10% of the tumor cells.

Although it is possible to establish the reference levels of staining, there exists considerable difficulty in establishing a consistent quality of staining of the samples themselves. This arises from a variety of different factors including inhomogeneous tissue material, the laborious and complex nature of the procedures, variability in reagent quality (including antibody/probe affinity and specificity), and the subjective nature of the interpretation carried out by the practitioner. Furthermore, other sources of variability in sample staining include the conditions under which tissue samples are collected, processed, and stored; variability in epitope retrieval procedures; and enzyme catalyzed chromogen precipitation.

As an attempt to solve these problems, it is known in the prior art to include reference sets of unstained tissues or cells with different levels of expression of the relevant antigen (or nucleic acid). The reference samples may comprise biopsy samples (e.g., tissue samples) from known diseased and normal individuals, or individuals which express antigen or relevant detectable nucleic acid at higher than normal levels but are not clinically diseased. Furthermore, tissue culture cells, which may be transfected with expression vectors to enable them to express the antigen or detectable nucleic acid at various levels, may also be used as reference standards. The reference sets may comprise slides with fixed and formalin embedded cells, but are otherwise unstained, and may be embedded in paraffin. The slides may then be processed in parallel with the sample to reveal the level and pattern of antigen (or detectable nucleic acid) staining. Finally, the sample may be compared to the reference set to determine whether protein or nucleic acid expression levels are diagnostically significant.

### Kits

Another embodiment of the invention includes a kit for use in the identification of the pERK target molecule in a pathological tissue sample. For example, the kit may include an antibody solution including a first or primary antibody that immunologically recognizes the target molecule, a second or secondary antibody which immunologically recognizes the first antibody and which is conjugated to a means of detection. The kit may also include a reagent to produce a detectable reaction product. Alternatively, the primary antibody, which recognizes the target molecule, may be labeled with the enzyme. The labeled antibody may be labeled with biotin rather than an enzyme. Then, in an additional step, enzyme-labeled avidin or streptavidin may be introduced to the sample and allowed to bind to the biotinylated antibody.

### EXAMPLES

The structures, materials, compositions, and methods described herein are intended to be representative examples of the invention, and it will be understood that the scope of the invention is not limited by the scope of the examples. Those skilled in the art will recognize that the invention may be practiced with variations on the disclosed structures, materials, compositions and methods, and such variations are regarded as within the ambit of the invention.

### Example 1. Immunohistochemical Staining of pERK Antibody

Tumor biopsy samples are collected at baseline before starting treatment and additional samples are collected at various time points during the treatment cycles. Samples are collected from the same tumor site. The tumor samples are paraffin-embedded prior to analysis by immunohistochemistry.

### A. Manual Staining Procedure

The slide sections are deparaffinize by placing the slides in citrate buffer (0.01 M, pH 6.0) that was heated to approximately 95°C and then placing the slides in a microwave on high for approximately 2-3 minutes. The container with buffer and slides was then placed into a preheated steamer for approximately 30 minutes. When complete, the slides were allowed to remain in the heated buffer and acclimate to room temperature for approximately 30 minutes. The slides were then rinsed in distilled water. Following this rinse, slides were blocked in a 1.5% hydrogen peroxide solution for 10 minutes, washed in distilled water, and then washed in PBS (0.01 M, pH 7.4) for approximately 5 minutes.

The slides were incubated in primary antibody [phospho-p44/42 MAPK (Thr202/Tyr204)] at a dilution of 1:50 for approximately 30 minutes. The negative control slides were incubated in corresponding IgG. The slides were rinsed in PBS for 5 minutes, and then rabbit HRP-labeled polymer (e.g., DAKO EnVision System, HRP (DAB), #K4011) was placed on the slides for 30 minutes. The slides were rinsed in PBS for 5 minutes. Then, DAB substrate chromagen was applied for 5 minutes, followed by a wash in water for 5 minutes. The slides were counterstained with filtered Hematoxylin for approximately 20 seconds. The slides were washed with warm water, dehydrated, and a coverslip was mounted with permount.

### B. Automated Staining Procedure

The slide sections are deparaffinize by placing the slides in citrate buffer (0.01 M, pH 6.0) that was heated to approximately 95°C and then placing the slides in a microwave on high for approximately 2-3 minutes. The container with buffer and slides was then placed into a preheated steamer for approximately 30 minutes. When complete, the slides were allowed to remain in the heated buffer and acclimate to room temperature for approximately 30 minutes. The slides were then rinsed in DAKO Wash Buffer (see, e.g., Wilhelm, et al., Cancer Res. 64:7099-7109, 2004, which is incorporated herein by reference in its entirety).

The slides were then stained utilizing an autostainer (e.g., DAKO Autostainer Universal Staining System, DakoCytomation, Inc., Carpinteria, CA). The slides were blocked in a 1.5% hydrogen peroxide solution for 10 minutes, and then incubated for 20 minutes with normal blocking serum. The slides were then washed in DAKO buffer, and incubated in primary antibody [phospho-p44/42 MAPK (Thr202/Tyr204)] at a dilution of 1:50 for approximately 30 minutes. The negative control slides remained in blocking serum. Following incubation in primary antibody, the slides were rinsed in DAKO buffer. The slides were then incubated for 30 minutes with diluted biotinylated secondary antibody solution, and then rinsed in DAKO buffer. The slides were incubated for 30 minutes with the Vectastain Elite ABC Reagent (e.g.,Vector PK-6101, Vector Laboratories, Burlingame, CA), and then rinsed with DAKO buffer. DAB substrate chromagen was then applied to the slides for 1 minute followed by a rinse with distilled water. The slides were counterstained with DAKO Automation Hematoxylin for 1 minute, rinsed in DAKO buffer, rinsed in distilled water, dehydrated, and a coverslip was mounted with permount.

### C. Evaluation of tissue sections stained with anti-phosplao-ERK antibody

The slides are evaluated qualitatively and semi-quantitatively. Qualitative assessment consists of assessing the staining intensity, identifying the positively staining cells and the intracellular compartments involved in staining, and evaluating the overall slide quality. Separate evaluations are performed on the tumor cells and the tumor cell stroma. Intensity of staining was graded based upon the following scale: Negative, ± (equivocal), 1+ (weak), 2+ (moderate), 3+ (strong), or 4+ (intense). The intensity of staining is determined by evaluation of the entire specimen. Semi-quantitative evaluation of target antigen expression is conducted using a grading system based upon the percentage of cells throughout the entire specimen expressing each target antigen as follows: 0-5% with positive target antigen expression = <5%; 6-25% = 1^{st} quartile (1Q); 26-50% = 2^{nd} quartile (2Q); 51-75% = 3^{rd} quartile (3Q); 76-100% = 4^{th} quartile (4Q). For quantitative evaluation, each tissue section is assessed using the Bioquant TCW98 image analysis system. For example, five (5) non-overlapping fields/tissue are selected and captured electronically using an Olympus BX-60 microscope and an Optronics DEI-750 color digital camera connected to a PC-based computer running the Bioquant software. The total numbers of individual immunostaining events per tissue area sampled per 20X field are measured. The data collected from these measurements are presented as a staining density per tissue section (i.e., number of staining events per unit area of tissue [# of stained cells/mm²]).

Patient best response is classified as outlined below:

Complete Response (CR): disappearance of all clinical and radiological evidence of tumor (both *target* and *non-target*).

Partial Response (PR): at least a 30% decrease in the sum of longest diameter (LD) of target lesions taking as reference the baseline sum LD.

Minor Response (MR): less than a 30% decrease in the sum of longest diameter (LD) of target lesions taking as reference the baseline sum LD.

Stable Disease (SD): steady state of disease. Neither sufficient shrinkage to qualify for PR nor sufficient increase to qualify for PD.

Progressive Disease (PD): at least a 20% increase in the sum of LD of measured lesions taking as references the smallest sum LD recorded since the treatment started or the appearance of one or more new lesions. Appearance of new lesions may also constitute progressive disease (also known as progression). In exceptional circumstances unequivocal progression of a non-measured lesion may be accepted as evidence of disease progression.

To determine if a correlation exists between levels and/or intensity of pERK staining and patient-response, the following plots and statistical analysis of the pERK data are executed using the R software **(www.r-project.org)** version 1.9.0 on a PC running Windows 2000. The plots are generated by first creating a survival object using the Surv function and then using survfit to fit this object to the time to progression data. The plot function is then used on the result of survfit. The p-value, representing the significance of the comparison between two groups of patients is calculated using the survdiff function with the optional parameter rho set to 1. Thus, the algorithm may be used to calculate p was the Peto and Peto modification of the Gehan-Wilcoxon rank sum test.

For the classification of patients for percent nuclei stained, patients with reported values of less than 5% are grouped together in the first class. All other patients are grouped together in the second class. For the classification of patients for maximum staining intensity, staining intensity is reported as a range. The upper bound of the range is designated the maximum staining intensity. Patients reported to have either negative staining or a maximum intensity of 1 are placed together in the first class. All other patients are grouped together in the second class.

As depicted in Figure 1, there appears to be a statistically significant correlation between pERK levels-measured as maximum staining intensity within the tumor-and time to progression. In other words, the higher the baseline pERK staining level in the tumor cells, the more likely that patient is to have a delayed time to progression. A trend toward a correlation was observed between pERK levels defined by percent of nuclei expressing pERK and time to progression. However, the p value of this trend (p = .073) falls just outside of the statistically significant range (p≤ 0.05). In addition, there was a statistically significant difference (p = 0.00093) between "responders" (Partial Response (PR) or patients with Stable Disease (SD)) and "non-responders" (Progressive Disease (PD)) in maximum staining intensity. The mean maximum staining intensity of "non-responders" on the 1+ to 4+ scale was 0.88+, whereas in "responders," the mean was 2.04+. Moreover, no non-responder had a maximum staining intensity greater than 1+.

Furthermore, statistical analysis demonstrates that for all tumor types combined, pERK levels are statistically significantly (p = 0.0024) higher in "non-responders" (patients with Progressive Disease) than "responders" (PR, MR, SD). Furthermore, patients with tumors that have higher levels of pERK show statistically significantly shorter time to tumor progression (TTP) (p < 0.05) and time to death (TTD) (p = 0.023).

The methods and materials described herein are intended to be representative examples of the invention, and it will be understood that the scope of the invention is not limited by the scope of the examples.

## Claims

1. A method to monitor the response of a patient being treated for cancer to the administration of a small molecule Raf kinase inhibitor, comprising the steps of:
a. determining the phosphorylation level of pERK in a first biological sample obtained from the patient prior to treatment with the small molecule Raf kinase inhibitor:
b. determining the phosphorylation level of pERK in at least a second biological sample obtained from the patient subsequent to the treatment with the small molecule Raf kinase inhibitor; and
c. comparing the phosphorylation level in the second biological sample with the phosphorylation level in the first biological sample; wherein the phosphorylation level of pERK -is assessed by immunohistochemistry, and wherein a change in the phosphorylation level of pERK proteins in the second biological sample compared to the phosphorylation level of pERK in the first biological sample indicates the efficacy of the treatment with the small molecule Raf kinase inhibitor.

2. The method of claim 1, wherein said cancer is selected from lung cancer, renal cancer, pancreatic cancer, liver cancer, gastrointestinal cancer, thyroid cancer, ovarian cancer, breast cancer, prostate cancer, and melanoma.

3. The method of claim 1, wherein said sample is a tumor biopsy.

4. A method for selecting a cancer patient responsive to a small molecule Raf kinase inhibitor, comprising the steps of:
a) determining the phosphorylation level of pERK -in a first biological sample obtained from a cancer patient, said patient having been administered a small molecule Raf kinase inhibitor;
b) comparing the phosphorylation level of pERK in the first biological sample with the phosphorylation level of pERK in a second biological sample obtained from a normal subject said normal subject having been administered a small molecule Raf kinase inhibitor; wherein the phosphorylation level of pERK is assessed by immunohistochemistry and a change in the phosphorylation level of pERK in the first biological sample compared to the phosphorylation level of pERK in the second biological sample is a prognostic of that patient's response to treatment with the small molecule Raf kinase inhibitor.

5. The method of claim 4, wherein the patient has been diagnosed with cancer is selected from lung cancer, renal cancer, pancreatic cancer, liver cancer, gastrointestinal cancer, thyroid cancer, ovarian cancer, breast cancer, prostate cancer, and melanoma.

6. The method of claim 4, wherein said sample is a tumor biopsy.

## Patentansprüche

1. Verfahren zum Überwachen der Antwort eines wegen Krebs behandelten Patienten auf die Verabreichung eines Kleinmolekül-Raf-Kinase-Inhibitors, umfassend die folgenden Schritte:
a. die Bestimmung des Phosphorylierungsausmaßes von pERK in einer ersten biologischen Probe, die von dem Patienten vor der Behandlung mit dem Kleinmolekül-Raf-Kinase-Inhibitor erhalten wurde;
b. die Bestimmung des Phosphorylierungsausmaßes von pERK in zumindest einer zweiten biologischen Probe, die von dem Patienten nach der Behandlung mit dem Kleinmolekül-Raf-Kinase-Inhibitor erhalten wurde; und
c. das Vergleichen des Phosphorylierungsausmaßes in der zweiten biologischen Probe mit dem Phosphorylierungsausmaß in der ersten biologischen Probe;
worin das Phosphorylierungsausmaß von pERK mittels Immunhistochemie untersucht wird und worin eine Veränderung des Phosphorylierungsausmaßes von pERK-Proteinen in der zweiten biologischen Probe im Vergleich zum Phosphorylierungsausmaß von pERK in der ersten biologischen Probe die Wirksamkeit der Behandlung mit dem Kleinmolekül-Raf-Kinase-Inhibitor zeigt.

2. Verfahren nach Anspruch 1, worin die Krebsart aus Lungenkrebs, Nierenkrebs, Pankreaskrebs, Leberkrebs, Magen-Darm-Krebs, Schilddrüsenkrebs, Eierstock-Krebs, Brustkrebs, Prostatakrebs und Melanomen ausgewählt ist.

3. Verfahren nach Anspruch 1, worin die Probe eine Tumorbiopsie ist.

4. Verfahren zur Auswahl eines Krebspatienten, der auf einen Kleinmolekül-Raf-Kinase-Inhibitor anspricht, umfassend die folgenden Schritte:
a. die Bestimmung des Phosphorylierungsausmaßes von pERK in einer ersten biologischen Probe, die von einem Krebspatienten erhalten wurde, dem ein Kleinmolekül-Raf-Kinase-Inhibitor verabreicht wurde;
b. das Vergleichen des Phosphorylierungsausmaßes von pERK in der ersten biologischen Probe mit dem Phosphorylierungsausmaß von pERK in einer zweiten biologischen Probe, die von einem normalen Individuum erhalten wurde, wobei das normale Individuum einen Kleinmolekül-Raf-Kinase-Inhibitor erhalten hatte;
worin das Phosphorylierungsausmaß von pERK mittels Immunhistochemie untersucht wird und eine Veränderung des Phosphorylierungsausmaßes von pERK in der ersten biologischen Probe im Vergleich zu dem Phosphorylierungsausmaß von pERK in der zweiten biologischen Probe eine Prognose bezüglich des Ansprechens dieses Patienten auf die Behandlung mit dem Kleinmolekül-Raf-Kinase-Inhibitor ermöglicht.

5. Verfahren nach Anspruch 4, worin bei dem Patienten Krebs diagnostiziert wurde, der aus Lungenkrebs, Nierenkrebs, Pankreaskrebs, Leberkrebs, Magen-Darm-Krebs, Schilddrüsenkrebs, Eierstock-Krebs, Brustkrebs, Prostatakrebs und Melanomen ausgewählt ist.

6. Verfahren nach Anspruch 4, worin die Probe eine Tumorbiopsie ist.

## Revendications

1. Procédé de surveillance de la réaction d'un patient sous traitement pour cancer à l'administration d'un inhibiteur à petites molécules de la Raf kinase, comprenant les étapes de :
a. détermination du taux de phosphorylation de pERK dans un premier échantillon biologique obtenu auprès du patient avant le traitement par l'inhibiteur à petites molécules de la Raf kinase ; et
b. détermination du taux de phosphorylation de pERK dans au moins un second échantillon biologique obtenu auprès du patient à la suite du traitement par l'inhibiteur à petites molécules de la Raf kinase ; et
c. comparaison du taux de phosphorylation dans le second échantillon biologique avec le taux de phosphorylation dans le premier échantillon biologique ; où le taux de phosphorylation de pERK est établi par immunohistochimie et où une modification dans le taux de phosphorylation des protéines pERK dans le second échantillon biologique par comparaison au taux de phosphorylation de pERK dans le premier échantillon biologique indique l'efficacité du traitement par l'inhibiteur à petites molécules de la Raf kinase.

2. Procédé selon la revendication 1, où ledit cancer est sélectionné parmi le cancer du poumon, le cancer du rein, le cancer pancréatique, le cancer hépatique, le cancer gastro-intestinal, le cancer de la thyroïde, le cancer ovarien, le cancer du sein, le cancer de la prostate et le mélanome.

3. Procédé selon la revendication 1, où ledit échantillon est une biopsie tumorale.

4. Procédé de sélection d'un patient cancéreux réagissant à un inhibiteur à petites molécules de la Raf kinase, comprenant les étapes de :
a) détermination du taux de phosphorylation de pERK dans un premier échantillon biologique obtenu auprès d'un patient cancéreux, ledit patient s'étant vu administré un inhibiteur à petites molécules de la Raf kinase ;
b) comparaison du taux de phosphorylation de pERK dans le premier échantillon biologique avec le taux de phosphorylation de pERK dans un second échantillon biologique obtenu auprès d'un sujet normal, ledit sujet normal s'étant vu administré un inhibiteur à petites molécules de la Raf kinase ; où le taux de phosphorylation de pERK est établi par immunohistochimie et une modification du taux de phosphorylation de pERK dans le premier échantillon biologique par comparaison au taux de phosphorylation de pERK dans le second échantillon biologique est un pronostic de la réaction de ce patient au traitement par l'inhibiteur à petites molécules de la Raf kinase.

5. Procédé selon la revendication 4, où il a été diagnostiqué chez le patient un cancer sélectionné parmi le cancer du poumon, le cancer du rein, le cancer pancréatique, le cancer hépatique, le cancer gastro-intestinal, le cancer de la thyroïde, le cancer ovarien, le cancer du sein, le cancer de la prostate et le mélanome.

6. Procédé selon la revendication 4, où ledit échantillon est une biopsie tumorale.
